# EUROPÄISCHE PATENTSCHRIFT

(11) **EP 0 620 727 B1**
(45) Veröffentlichungstag und Bekanntmachung des Hinweises auf die Patenterteilung: **16.04.1997**
(21) Anmeldenummer: 93902033.5
(22) Anmeldetag: 18.01.1993
(51) Int. Cl.: A61K 9/16, A61K 9/20

(54) **PFLANZENWIRKSTOFFEXTRAKT ENTHALTENDE KRYOPELLETIERTE FORMKÖRPER UND IHRE PHARMAZEUTISCHE ODER KOSMETISCHE VERWENDUNG**
CRYOPELLETED MOULDED BODIES, CONTAINING PLANT ACTIVE SUBSTANCE EXTRACT AND THE PHARMACEUTICAL OR COSMETIC USE OF SUCH PELLETS
ELEMENTS MOULES CRYOPELLETES, CONTENANT DES EXTRAITS DES SUBSTANCES ACTIVES DE PLANTES ET LEUR APPLICATION PHARMACEUTIQUE OU COSMETIQUE

(30) Priorität: 17.01.1992 DE 4201172; 17.01.1992 DE 4201179; 30.04.1992 US 876866; 30.04.1992 US 876876
(43) Veröffentlichungstag der Anmeldung: 26.10.1994
(73) Patentinhaber: ALFATEC-PHARMA GmbH, 69120 Heidelberg (DE)
(72) Erfinder: WUNDERLICH, Jens-Christian, D-6900 Heidelberg (DE); SCHICK, Ursula, D-69198 Schriesheim (DE); WERRY, Jürgen, D-6700 Ludwigshafen (DE); FREIDENREICH, Jürgen, D-6905 Schriesheim (DE)
(74) Vertreter: KUHNEN, WACKER & PARTNER
(86) Internationale Anmeldenummer: DE9300037
(87) Internationale Veröffentlichungsnummer: WO9313754

(56) Entgegenhaltungen:
- BE-A- 770 604
- DE-A- 454 386
- FR-A- 1 259 081
- FR-A- 1 397 583
- GB-A- 242 323
- J.E.F. REYNOLDS 'MARTINDALE THE EXTRA PHARMACOPOEIA' 1989 , THE PHARMACEUTICAL PRESS , LONDON
- CHEMICAL ABSTRACTS, vol. 113, no. 13, 1990, Columbus, Ohio, US; abstract no. 114127d,

## Beschreibung

Die vorliegende Erfindung betrifft Pflanzenwirkstoffextrakt enthaltende kryopelletierte Pellets bzw. Vollkugeln, welche durch eine Dispersion des Pflanzenextraktes in einer Matrix, die vorwiegend aus einem Gerüstbildner aus einem hydrophilen Makromolekül besteht, gekennzeichnet sind.

Die Erfindung betrifft weiterhin ein schonendes Verfahren zur Herstellung solcher Pellets oder Vollkugeln, wie ihre pharmazeutische, perorale oder kosmetische Anwendung.

ALs bevorzugter Pflanzenextrakt wird Aloe Vera Saft eingesetzt.

Pflanzenextrakte im Sinne der vorliegenden Erfindung sind direkt aus der Pflanze gewonnene Frischpflanzensäfte, Preßsäfte aus Frischpflanzen, sowohl in ursprünglicher Konzentration als auch in aufkonzentrierter Form, filtriert und unfiltriert, hydrophile (wäßrige oder alkoholische, z.B. Ethanol-oder 1,2-Propylenglykol-Auszüge) Extrakte, wie z.B. homöopathische Urtinkturen, Fluidextrakte, Mazerate, lipophile Extrakte (wie z.B. Knoblauchöl), ätherische Öle, Gesamtextrakte oder speziell standardisierte Extrakte (z.B. auf einen bestimmten Gehalt von Flavonglykosiden eingestellt), ätherische-Öl-Auszüge, einzeln isolierte Pflanzeninhaltsstoffe (wie z.B. Rutin), synthetische Analoga (wie z.B. Parfümöle, Campher, Thymol, Vanillin), derivatisierte Pflanzeninhaltsstoffe (wie z.B. Aglyka).

In Einzelfällen können auch Trockenextrakte zur Anwendung kommen bzw. der aus einem Trockenextrakt im entsprechenden Lösungsmittel wieder aufgelöste Extrakt oder daraus hergestellte Dekokte. Ebenso können pulverisierte Drogenbestandteile (z.B. Blätter, Wurzeln, Kraut), verarbeitet werden.

Pflanzenextrakte sind als Naturstoffe in vielen Fällen empfindlich gegen äußere Einflüsse wie Licht, Oxidation durch Luftsauerstoff, Wärme, pH-Einflüsse in Lösungen oder mikrobiellen Befall. Von vielen pflanzlichen Wirkstoffen ist bekannt, daß die optimale Wirksamkeit nur durch den ursprünglich aus der Pflanze oder deren Teilen gewonnenen frischen Saft (z.B. Echinacea-Preßsaft oder Aloe Vera Saft) gegeben ist. Jede Maßnahme zur Konservierung wie Trocknung durch Wärme, chemische Konservierung, Wärmehehandlung zur Konservierung etc. beeinträchtigen die empfindlichen Pflanzeninhaltsstoffe in ihrer chemischen Struktur und somit in ihrer Wirksamkeit. In den meisten Fällen ist beim Einsatz von Frischpflanzensäften eine Konservierung gegen mikrobiellen Befall unumgänglich, um zumindest eine begrenzte Haltbarkeit zu erreichen.

Hinzu kommt, daß die Trockensubstanz in solchen Frischpflanzensäften sehr gering ist und man eine große Menge Wasser transportiert bzw. lagert.

Oxidationsempfindliche lipophile Pflanzenauszüge wie Vitamin E oder Knoblauchöl sind in unveränderter Form schwierig zu lagern und müssen meist sofort verarbeitet werden.

Ätherische Öle sind leicht flüchtig und als Flüssigkeiten schwierig zu handhaben.

Aloe Vera (Aloe Barbadensis Miller; Synonyme: Aloe Vera Tournefort ex Linne, Aloe Vulgaris Lammarck) ist seit alters her in der traditionellen Volksmedizin derjenigen Regionen bekannt, in der diese zur Familie der Liliaceen gehörende Pflanze wild wächst.

Äußerlich angewendet hat der gelartige Pflanzensaft beispielsweise die Eigenschaft, die Wundheilung zu fördern, antibiotisch zu wirken oder einen weichmachenden Effekt auf der Haut zu besitzen.

Innerlich kann Aloe Vera Saft bei der Behandlung von Magenerkrankungen und Störungen des Gastrointestinaltraktes angewendet werden. Ebenso wird über antiinflammatorische Eigenschaften berichtet.

Diese ursprünglichen Erkenntnisse führten dazu, daß Aloe Vera heute in großen Plantagen in Mittel-, Süd- und in Teilen Nordamerikas angepflanzt wird. Der in den Blättern enthaltene Saft wird vor Ort in einem arbeitsintensiven Prozeß gewonnen und anschließend unter möglichst milden Bedingungen aufkonzentriert. Die Konzentration der Inhaltsstoffe in dem frisch gewonnenen Saft liegt zwischen 0.3 und ca. 1%. Es werden sowohl frisches Aloe Vera-Filet, wäßrige Konzentrate bzw. sprüh- oder gefriergetrocknete Ware gehandelt. Die Qualitätsunterschiede handelsüblicher Produkte sind hinsichtlich Stabilität und Zusammensetzung erheblich und hängen wesentlich von der eingesetzten Herstellungstechnologie ab.

Wäßrige Konzentrate oder der Saft aus den Blättern werden heute erfolgreich bei Hautaffektionen (z.B. Verbrennungen durch Hitzeeinwirkung, UV- und Röntgenstrahlen) Verätzungen, Wunden, Magenerkrankungen oder Parodontose eingesetzt. Die pharmakologische Wirkung ist dabei scheinbar nur der Gesamtheit aller Inhaltsstoffe zuzuschreiben. Der Nachweis über die Wirkung von Einzelkomponenten wird z.Zt. intensiv erforscht.

Da bisher jedoch keine unerwünschten Nebenwirkungen von Aloe Vera Saft bekannt geworden sind, wird seit Jahren dieses Naturprodukt in Cremes, Feuchthalteemulsionen, Sonnenschutzmitteln oder zur innerlichen Anwendung angeboten.

Grundlegende Schwierigkeiten treten bei der Haltbarkeit bzw. Lagerstabilität des wässrigen Pflanzengels auf. Das flüssige Produkt ist trotz Konservierung wärme- und pH-instabil, sauerstoffempfindlich und darüberhinaus gegen mikrobiellen Befall sehr anfällig.

Ein Transport von frischem Aloe Vera Saft ist aufgrund des großen Flüssigkeitsvolumens - man befördert 90-99% Wasser - und der genannten Instabilitäten schwierig und kostenintensiv. Bis zum Einsatz in der Produktion muß der Saft außerdem gekühlt werden. Die Herstellungsmethoden mit den Verfahrensschritten Eingangswäsche der Blätter, Gewinnung der Filets, Homogenisierung, Reinigung und Filtration, Aufkonzentrierung und Trocknung können bei ungeeigneter Technik zu Veränderungen der Inhaltsstoffe und bakterieller Kontamination des jeweiligen Endproduktes führen.

Durch Sprüh-oder Gefriertrocknung erhaltene Aloe Vera-Pulver sind aufgrund mangelnder Benetzbarkeit bzw. der Gefahr von Verklumpungen nur schwierig in kaltem Wasser wieder aufzulösen. Darüberhinaus sind gefriergetrocknete Produkte hygroskopisch, die bei unsachgemäßer Lagerung leicht zum Verkleben neigen. Handelsüblichen Produkten, die sich gut in Wasser auflösen lassen, sind oft Tenside zugesetzt, die in der Kosmetik unerwünscht sind.

Die JP, A, 2 135 052 = C.A. 113 (1990) 11 41 27d beschreibt Instant-Nahrungsmittel in körniger Form. Hierbei werden Öl und/oder öllösliche Substanzen in wässrigen Lösungen, die Exzipienten enthalten, dispergiert und die Lösung in flüssigen Stickstoff, verflüssigte Luft oder CO₂ getropft. Als Exzipient wird unter anderen Neosoft 200 GS genannt, das Gelatine enthält.

Der Erfindung liegt daher die Aufgabe zugrunde, unkonservierte, lagerstabile, konzentrierte, feste oder halbfeste Formen von Pflanzenextrakten, insbesondere von Aloe Vera Saft, anzubieten, die sich ohne Probleme wiederauflösen lassen und deren pharmakologische wie kosmetische Eigenschaften im Vergleich zum nativen Pflanzensaft unverändert erhalten bleiben.

Diese Aufgabe wird erfindungsgemäß durch Pflanzenwirkstoffextrakt enthaltende kryopelletierte Pellets gelöst, die gekennzeichnet sind durch eine Dispersion des Pflanzenextraktes in einer Matrix, die vorwiegend einen Gerüstbildner aus einem hydrophilen Makromolekül umfaßt, der aus der folgenden Gruppe ausgewählt ist:
Kollagen, Gelatine, fraktionierte Gelatine, Kollagenhydrolysate, Gelatinederivate, Pflanzenproteine, Pflanzenproteinhydrolysate, Elastinhydrolysate; und Mischungen der vorgenannten Stoffe.

Diese Aufgabe wird ferner durch ein Verfahren zur Herstellung von Pflanzenwirkstoffextrakt enthaltenden kryopelletierten Pellets gelöst, das dadurch gekennzeichnet ist, daß man
a) einen Gerüstbildner aus hydrophilen Makromolekülen ausgewählt aus der Gruppe bestehend aus: Kollagen, Gelatine, fraktionierter Gelatine, Kollagenhydrolysaten, Gelatinederivaten Pflanzenproteinen, Pflanzenproteinhydrolysaten, Elastinhydrolysaten; sowie deren Mischungen, mit Pflanzenwirkstoffextrakten vermischt, welche ausgewählt werden aus der Gruppe bestehend aus: hydrophilen flüssigen Pflanzenextrakten, wäßrigen Extrakten, alkoholischen Extrakten; sowie deren Mischungen; und
b) die erhaltene Mischung aus Gerüstbildner und Pflanzenextrakt in eine tiefkalte inerte Flüssigkeit eintropft und damit Formkörper formt.

Die Formkörper können bei Bedarf getrocknet werden. Einzig ist darauf zu achten, daß keine Unverträglichkeiten von Matrixsystemen und Wirkstoff auftreten.

Bevorzugte Ausführungsformen der Erfindung sind in den Unteransprüchen beschrieben und beansprucht. Pflanzenwirkstoffextrakte bzw. daraus gewonnene Auszüge oder Einzelstoffe können z.B. sein:

### Flavonoide und ihre Aglyka:

Rutin, Quercetin, Diosmin, Hyperosid, (Neo)hesperidin, Hesperitin, Ginkgo Biloba (z.B. Ginkoflavonglykoside), Crataegus-Extrakt (z.B. oligomere Procyanidine), Buchweizen (z.B. Rutin), Sophora japonica (z.B. Rutin), Birkenblätter (z.B. Quercetinglykoside, Hyperosid und Rutin), Holunderblüten (z.B. Rutin), Lindenblüten (z.B. ätherisches Öl mit Quercetin und Farnesol), Johanniskrautöl, (z.B. Olivenölauszug), Calendula, Arnika (z.B. ölige Auszüge der Blüten mit ätherischem Öl, polare Auszüge mit Flavonoiden), Melisse (z.B. Flavone, ätherisches Öl),

### Ätherische Öle:

Salbei (z.B. ätherisches Öl mit Thymol), Anis (z.B. ätherisches Öl mit trans-Anethol), Nelkenöl (z.B. ätherisches Öl mit Eugenol), Kamille (z.B. Chamazulen, alpha-Bisabolol), Myrtol: (Limonen, alpha-Pinen, Cineol), Pfefferminzöl (z.B. Öl mit Menthol), Kümmel (z.B. Öl mit Carvon), Latschenkiefer (z.B. Öl mit alpha-Pinen),
Wacholder, Rosmarin, Eukalyptusöl, Lavendel, Fichtennadelöl, Bergamotteöl, Citrusöl, Melisse, Majoran, Thymian, Basilikum (Stomachika bzw. Gewürze), Fenchel

### Fette Öle,

z.B. Weizenkeimöl und daraus isoliertes Vitamin E, Nachtkerzenöl (z.B. gamma-Linolensäure), Pflanzenlecithine (z.B. Sojalecithin), aus Pflanzen isolierte Sphingolipide/Ceramide

### Immunstimulantien:

Echinacea purpurea (z.B.alkoholische Auszüge, Frischpflanzensaft, Preßsaft), Eleutherokokkus senticosus

### Alkaloide:

Rauwolfia (z.B. Prajmalin),Immergrün (z.B.Vincamin)

### Weitere Phytopharmaka:

Aloe, Roßkastanie (z.B. Aescin), Knoblauch (z.B. Knoblauchöl), Ananas (z.B. Bromelaine), Ginseng (z.B. Ginsenoside), Mariendistelfrüchte (z.B. auf Silymarin standardisierter Extrakt), Mäusedornwurzel (z.B. Ruscogenin), Baldrian (z.B. Valepotriate, Tct. Valerianae), Kava-Kava (z.B. Kavalactone), Hopfenblüten (z.B. Hopfenbitterstoffe), Extr. Passiflorae, Enzian (z.B. ethanol. Extrakt), anthrachinonhaltige Drogenauszüge, z.B. aloinhaltiger Aloe Vera-Saft, Pollenextrakt, Algenextrakte, Süßholzwurzelextrakte, Palmenextrakt, Galphimia (z.B.Urtinktur), Mistel (z.B. wäßrigethanol. Auszug), Phrtosterole (z.B. beta-Sitosterin), Wollblumen (z.B. wäßrig-alkohol. Extrakt), Drosera (z.B. Likörweinextrakt), Sanddornfrüchte (z.B. daraus gewonnener Saft oder Sanddornöl), Eibischwurzel, Primelwurzelextrakt, Frischpflanzenextrakte aus Malve, Beinwell, Efeu, Schachtelhalm, Schafgarbe, Spitzwegerich (z.B. Preßsaft), Brennessel, Schöllkraut, Petersilie.

Pflanzenextrakte aus Norolaena lobata, Tagetes lucida, Tecoma siems, Momordica charantia.

Allgemein wird der Pflanzenwirkstoffextrakt in der Regel ausgewählt aus der Gruppe bestehend aus: feste Pflanzenextrakte, flüssige Pflanzenextrakte, hydrophile Pflanzenextrakte, lipophile Pflanzen-extrakte, einzelne Pflanzeninhaltsstoffe; sowie deren Mischungen.

Im allgemeinen wird in der Literatur von Aloe Vera Saft, Aloe Vera Gel und von Aloe Vera Extrakten gesprochen. Im Sinne der Erfindung ist der verwendete Begriff "Aloe Vera Saft" allgemein sowohl als der native, direkt aus dem Blatt gewonnene Saft, der filtrierte bzw. gereinigte Saft, der unter schonenden Bedingungen konzentrierte Saft als auch der aus einem Trockenextrakt wiederaufgelöste Saft zu verstehen. Zur innerlichen Anwendung kann auch das vollständige Blatt, Blattbestandteile und Blüten in homogenisierter Form verwendet werden. Für spezielle Anwendungen können auch einzelne Inhaltsstoffe geeignet sein.

Die erfindungsgemäßen Pellets stellen runde, einheitliche Formkörper mit Durchmessern im üblichen Bereich von 0,8 - 2 mm dar. Darüberhinaus sind erfindungsgemäß Größen von 0,2 - 0,8 und von 2 - 12 mm herstellbar. Pellets ab einem Durchmesser von 2 mm werden in der vorliegenden Erfindung als Vollkugeln bezeichnet und eignen sich als einzeldosierte Arzneiform (single unit dosage form).

Überraschenderweise weisen die Pellets eine hohe Festigkeit bei geringem Abrieb (Friabilität) auf. Sie sind lagerstabil, gut dosierbar und fallen, bedingt durch den besonderen Herstellungsprozeß, als freifließendes Gut an. Sie können Pflanzenextrakt(e), berechnet als Feststoffanteil in Konzentrationen von 0,1-98 % (Gewichtsprozente), bevorzugt 0,1-60% enthalten.

Erstaunlicherweise ändert sich weder Art noch Zusammensetzung der Inhaltsstoffe der nativen Pflanzeninhaltsstoffe durch die erfindungsgemäßen Pellets. Als Kälteverfahren ist das erfindungsgemäße Verfahren eine sehr schonende Form der Aufarbeitung Die Pflanzenextrakt(e) enthaltenden Pellets können je nach Herstellung als Lyophilisat oder als feste bzw. gelförmige Pellets vorliegen.

Durch Überführung in Pelletform mit dem erfindungsgemäßen Matrixsystemen werden Konservierungsstoffe bzw. eine Wärmebehandlung zum Konservieren überflüssig.

Ein pelletierter Frischpflanzensaft ist lagerstabil. Ist z.B. eine Lösungsmittelextraktion aus Gründen der Stabilität notwendig, so kann diese entfallen.

Weiterhin ist die Überführung von Flüssigkeiten in die feste Form möglich (Ätherische Öle oder Fette Öle). Dies verbessert die Lagerstabilität, Transportmöglichkeit und die Handhabbarkeit von solchen Stoffen.

Die erfindungsgemäß konventionell getrockneten Pellets mit oder ohne Weichmacherzusätze sind an ihrem charakteristischen, unverwechselbaren Aussehen gut zu erkennen: Sie sind transparent oder opak glänzend.

Das erfindungsgemäße Produkt kann sowohl zu pharmazeutischen Zwecken, zur innerlichen Anwendung bzw. kosmetischen Zwecken direkt eingesetzt werden.

Zu pharmazeutischen Zwecken können die Pellets als Multiple Unit Dosage Forms als Granulat in Beutein bzw. in Hartgelatinekapseln abgefüllt werden, weiterhin als Trinkgranulat zum Herstellen von Trinklösungen (z.B. Instanttees) sowie als Single Unit Dosage Forms, d.h. einzelndosierte Pellets z.B. abgefüllt in geeignete Behälter, Blister oder in Dosierspendern zur Einzelentnahme konfektioniert werden. Eine weitere single unit form sind aus gefriergetrockneten Pellets gepreßte Tabletten, die sich schnell auflösen.

Für kosmetische Anwendungen ist es erfindungsgemäß besonders vorteilhaft, Pflanzenproteine oder deren Hydrolysate, lösliches Kollagen, Gelatine, Kollagenhydrolysate, Elastin oder Elastinhydrolysate als Trägermaterialien der Formkörper zu verwenden.

Gelatine ist ein aus kollagenhaltigem Material gewonnenes Skleroprotein, das je nach Herstellungsprozeß unterschiedliche Eigenschaften hat. Sie besteht im wesentlichen aus vier Molekulargewichtsfraktionen, die die physikalisch-chemischen Eigenschaften in Abhängigkeit vom Molekulargewicht und prozentualem Gewichtsanteil beeinflussen. Je höher z.B. der Anteil Mikrogel (107 bis 108 D) liegt, desto höher ist auch die Viskosität der wäßrigen Lösung. Handelsübliche Sorten enthalten bis zu 15 Gewichtsprozent. Die Fraktionen der alpha-Gelatine und deren Oligomere (9,5 x 104 / 105 bis 106 D) sind entscheidend für die Gelfestigkeit und liegen üblicherweise zwischen 10 und 40 Gewichtsprozent. Molekulargewichte unterhalb der alpha-Gelatine werden als Peptide bezeichnet und können in herkömmlichen Gelatinequalitäten (niedrigbloomig) bis zu 80 Gewichtsprozent betragen.

Gelatine besitzt ein temperatur- und konzentrationsabhängiges Sol-Gel-Umwandlungsverhalten, das von der molekularen Zusammensetzung abhängig ist. Als Konventionsmethode für das Gelbildungsvermögen wird die Bloomzahl angegeben. Niedrige Handelsqualitäten beginnen bei 50 Bloom, hochbloomige Sorten liegen bei etwa 300 Bloom.

Fraktionierte Gelatine stellt den Spezialfall von Gelatine dar und wird durch spezielle Herstellungstechniken, wie z.B. Ultrafiltration aus herkömmlicher Gelatine gewonnen. Die Zusammensetzung kann z.B. durch Entfernung von Peptiden (MG < 9,5 x 104 D) oder durch Mischungen aus Einzelfraktionen wie z.B. alpha-Ketten, dimeren und trimeren Ketten oder Mikrogel varriiert werden.

Kollagen in nativer Form ist wasserunlöslich. Durch spezielle Herstellungsverfahren gibt es heute lösliche Kollagentypen.

Gelatinederivate sind chemisch veränderte Gelatinen, wie z.B. succinylierte Gelatine, die auch als Plasmaexpander bekannt sind.

Unter Kollagenhydrolysat wird ein von Kollagen oder Gelatine druckhydrolytisch oder enzymatisch gewonnenes Produkt ohne Gelbildungsvermögen verstanden. Die Molekulargewichtszusammensetzung kann herstellungsbedingt zwischen einigen Hundert D bis unterhalb von 9,5 x 104 D liegen. Kollagenhydrolysate sind kaltwasserlöslich.

Diese Stoffe biogenen Ursprungs zeichnen für die äußerliche Anwendung sich nicht nur durch eine gute Hautverträglichkeit aus, sie lassen sich auch besonders gut in Salben, Cremes und Emulsionen einarbeiten. Dabei zeigen sie ihre besondere Eigenschaft, zu einem gewissen Teil emulgierend und emulsionsstabilisierend zu wirken. So kann beispielsweise der Einsatz von größeren Mengen hautreizender Tenside weiter reduziert werden, was zur Hautverträglichkeit, die von pharmazeutischen Zubereitungen z.B. zur Wundbehandlung bzw. von modernen Kosmetika gefordert wird, beiträgt. Gelatine und Kollagenhydrolysate sind pharmazeutisch anerkannte Hilfsstoffe, die auch in der kosmetischen Industrie bevorzugt eingesetzt werden.

Neuentwickelte Produkte stellen die Pflanzenproteine und deren Hydrolysate dar, die in ihren Eigenschaften weitgehend den Kollagenhydrolysaten entsprechen. Sie werden vorzugsweise aus Weizen und Soja gewonnen und besitzen beispielsweise Molekulargewichte von 200.000-300.000 D bzw. 1.000-10.000 D.

Bei Verwendung von Pflanzenproteinen, Pflanzenproteinhydrolysaten bzw. von Kollagenhydrolysaten (kaltwasserlösliche Gelatinen) oder Gelatinen mit einem Maximum der Molekulargewichtsverteilung von einigen Hundert D bis unterhalb von 105 D bildet das lyophilisierte Trägermaterial der erfindungsgemäßen Formkörper überraschenderweise ein hochporöses Netzwerk mit stabiler mechanischer Eigenschaft aus.

Elastinhydrolysate werden enzymatisch aus Elastin gewonnen und bestehen aus einer einzigen Polypeptidkette mit einem hohen Anteil an nichtpolaren Aminosäuren. Aufgrund ihrer hydrophoben Eigenschaften können sie in lipophilen Systemen verwendet werden. Elastinhydrolysate weisen ein Molekulargewicht von ca. 2.000 - 3.000 D auf und sind auf der Haut stark filmbildend.

Die schnelle Auflösung der beschriebenen Pelletrezepturen ist vorteilhaft für Instantanwendungen wie z.B. Instanttees, Instantsäfte (z.B. Hustensaft) oder unkonservierte Instantcremes einsetzbar.

Die anerkannte Heilwirkung von Frischpflanzensäften, z.B. von Aloe Vera Saft, bei innerlicher Anwendung (health care) läßt sich durch die erfindungsgemäßen Pellets vorteilhaft in Form einer unkonservierten Instantzubereitung verbessern. Wird z.B. ein Frischpflanzensaft mit einer schnell auflösenden Matrix cryopelletiert, so erhält man lagerstabile Pellets, die man (z.B. in Beutel abgefüllt) in Wasser oder Fruchtsäften, Milch oder anderen Getränken innerhalb weniger Sekunden vollständig auflösen kann. Vorteilhaft sind auch komplette Fertigdrinks erfindungsgemäß herstellbar, bestehend aus Frischpflanzensaft, einer Matrixmasse aus Proteinen biogenen Ursprungs (z.B.Kollagenhydrolysaten, Weizenproteinen) und natürlichen Gerüstbildnern, Fruchtsaftextrakt, Honig und anderen natürlichen Bestandteilen. Bestandteile der Matrix wie z.B. Gelatine können unangenahmen Geschmack maskieren, Glycerol und Sorbitol können als zahnfreundliche Süßungsmittel fungieren.

Liegen die erfindungsgemäßen Pellets in nicht lyophilisierter Form vor, sondern in fester oder halbfester Form, können sie vorteilhaft aus sol-gel-bildenden hydrophilen Makromolekülen aufgebaut sein wie z.B. Gelatine oder fraktionierter Gelatine mit einem Maximum der Molekulargewichtsverteilung oberhalb 105 D, wobei die Konsistenz in direkter Abhängigkeit von Art und Konzentration des Weichmacherzusatzes abhängt.

Solche weichmacherhaltigen Pellets eignen sich hervorragend zur Überführung von ätherischen Ölen in eine feste und damit leicht zu verarbeitenden Form.

Insbesondere halbfeste Pellets können in der Matrixmasse so eingestellt werden, daß sie nach Applikation schmelzen bzw. sich auflösen. Für die äußerliche Anwendung sowohl im pharmazeutischen als auch im kosmetischen Bereich ist dabei die hautfreundliche Wirkung der aus natürlichen Stoffen aufgebauten Matrix ist von Vorteil.

Im folgenden wird das Verfahren zur Herstellung der erfindungsgemäßen Pellets näher beschrieben.

Weitere Ausführungen hierzu sind in den im folgenden aufgelisteten parallelen internationalen (PCT)-Anmeldungen enthalten. Die Inhalte dieser parallelen PCT-Anmeldungen, am selben Tage beim Deutschen Patentamt von denselben Erfindern und Anmeldern eingereicht:
WO93/13757
WO93/13761
WO93/13753
der, wie die älteren PCT-Anmeldungen:
WO 93/10768 WO93/10764
WO93/10761 WO93/10760
WO93/10762 WO93/10771
WO93/10769 WO93/10770
WO93/10766 WO93/10767

Liegt ein wäßriger, alkoholischer oder wäßrig/alkoholischer Extrakt vor, läßt sich das erfindungsgemäße Verfahren zur Herstellung von Pflanzenwirkstoffextrakt enthaltende Pellets mit den folgenden zwei Verfahrensschritten beschreiben:
a) Man vermischt einen Gerüstbildner, in fester oder gelöster Form, aus hydrophilen Makromolekülen aus der Gruppe: Kollagen, Gelatine, fraktionierte Gelatine, Kollagenhydrolysate, Gelatinederivate, Pflanzenproteine, Pflanzenproteinhydrolysate, Elastinhydrolysate mit flüssigem, hydrophilen (wäßrigen, alkoholischen oder wäßrig/alkoholischen) Pflanzenextrakt.
b) Man tropft die erhaltene Mischung aus Gerüstbildner und flüssigem hydrophilen Pflanzenextrakt in ein tiefkaltes inertes verflüssigtes Gas ein und formt damit Formkörper.

Unter Formkörper wird im Sinne der Erfindung ein solcher verstanden, der ausgewählt ist aus der Gruppe bestehend aus: Pulver, Granulate, Pellets, Aggregate, die im wesentlichen symmetrisch ausgebildet sind.

In der Beschreibung der Erfindung werden die Eigenschaften, Herstellung und Verwendung anhand von runden Pellets bevorzugt dargestellt.

Jedoch kann der Fachmann auch andere Formkörper aus der Gruppe bestehend aus: Pulver, Granulate, im wesentlichen symmetrisch ausgebildete Aggregate vorteilhaft zur Herstellung, insbesondere von Arzneiformen, einsetzen.

Werden als Gerüstbildner beisielsweise kaltwasserlösliche kollagen- oder Pflanzenproteinhydrolysate eingesetzt, so kann ohne Anwendung von Wärme, d.h. am schonendsten gearbeitet werden.

Bei einer Ausführungsform des unter a) beschriebenen Verfahrensschrittes stellt man eine tropffähige Masse, vorwiegend bestehend aus hydrophilen Makromolekülen als Gerüstbildner, insbesondere Pflanzenproteine, Pflanzenproteinhydrolysate, Kollagen, Gelatine, fraktionierter Gelatine, Kollagenhydrolysaten, Elastinhydrolysaten oder Gelatinederivaten und wäßrigem, alkoholischem oder wäßrig/alkoholischem Pflanzenextrakt her.

Zunächst löst man entweder in dem frisch gewonnenen oder dem bereits aufkonzentrierten, flüssigen wäßrigen, alkoholischen oder wäßrig/alkoholischen Pflanzenextrakt den gewünschten Gerüstbildner, insbesondere Pflanzenproteine, Pflanzenproteinhydrolysate, Kollagen, Gelatine, fraktionierte Gelatine, Gelatinederivate, oder Kollagenhydrolysate auf oder mischt ihn in bereits gelöster Form mit dem Pflanzenextrakt, wobei die Art und Menge des eingesetzten Gerüstbildners und gegebenenfalls der Zusatz von weiteren Hilfsstoffen vom späteren Verwendungszweck der Pellets abhängt. Die Konzentration des Trägermaterials kann beispielsweise von 0,5 bis 60% (g/g), bevorzugt 0,5 bis 30% (bezogen auf die zu verarbeitende Masse) variieren. Die Anwendung von Wärme im Temperaturbereich von 30°C bis 45°C kann z.B. beim Einsatz von Gelatine erforderlich sein, um diese in die Solform zu überführen.

Weiterhin können Zusätze aus der Gruppe: Albumin, Agar-Agar, Gummi arabicum, Pektine, Traganth, Xanthan, natürliche sowie modifizierte Stärken, Dextrane, Dextrine, Maltodextrin, Chitosan, Alginate, Cellulosederivate, Polyvinylpyrrolidon, Dextran, Zucker, Glycin, Lactose, Mannit, Polyacrylsäure, Polymere aus Methacrylsäure, Polymere aus Methacrylsäureestern; sowie deren Mischungen in 1- 50%iger Konzentration zugefügt werden.

Zu dieser Grundmasse können weitere, für kosmetische, innerliche bzw. pharmazeutische Anwendung geeignete Hilfs- und Trägerstoffe, wie z.B. zusätzliche Gerüstbildner, die unten stehend näher beschrieben werden, Weichmacher, wie z.B. Glycerol oder Sorbit, Füllstoffe, wie z.B. Lactose, Dispergiermittel, wie z.B. Dinatriumphosphat, pH-Korrigentien, wie z.B. Dinatriumcitrat, Emulgatoren, wie z.B. Lecithin, Stabilisatoren, wie z.B. Ascorbinsäure, Kosolventien, wie z.B. Polyethylenglycol, natürliche Farbstoffe, wie z.B. Carotinoide, aromatisierende Stoffe oder Geschmackskorrigentien, wie z.B. Fruchsaftkonzentrate, zugesetzt werden.

In einer weiteren Verfahrensvariante können der Matrix Zusatze von Weichmachern von 1-50% (bezogen auf die zu verarbeitende Masse) ausgewählt aus der Gruppe bestehend aus: Glycerol, Propylenglykol, Polyethylenglykole, Triacetin, Sorbitol, Sorbitangemische, Sorbitollösungen, Glucosesirup und andere Polyole bzw. Zuckeralkohole; und deren Mischungen beigefügt sein.

Weiterhin kann es technologisch von Vorteil sein, neben dem Gerüstbildner aus hydrophilen Makromolekülen auch andere gerüstbildende Substanzen zur Rezepturmasse zuzusetzen.

Als zusätzliche Gerüstbildner können eingesetzt werden: Albumine, Agar-Agar, Gummi arabicum, Pektine, Traganth, Xanthan, natürliche sowie modifizierte Stärken, Dextrane, Dextrine, Maltodextrin, Chitosan, Alginate, Cellulosederivate, Zucker wie z.B. Saccharose, Glycin, Lactose, PVP (Polyvinylpyrrolidon), Mannit und Kombinationen der vorgenannten Stoffe untereinander, insbesondere aber Mannit.

Bei Pflanzenwirkstoffextrakten, die extrem thermolabil sind, ermöglicht die Erfindung in einer weiteren Ausführungsform überraschenderweise Formkörper mit den erfindungsgemäßen Eigenschaften zur Verfügung zu stellen, die unter ausschließlich kalten Bedingungen hergestellt sind. Bei dieser Vorgehensweise verwendet man eine Matrix aus einem hydrophilen Makromolekül ausgewählt aus der Gruppe bestehend aus: Pflanzenproteine, Pflanzenproteinhydrolysate, Elastinhydrolysate, Kollagenhydrolysate, kaltwasserlösliche Gelatine, Gelatinederivate; und Mischungen der vorgenannten Stoffe mit einem Maximum der Molekulargewichtsverteilung unterhalb 10⁵ D.

In einer Ausführungsform der Erfindung können Zusätze von Stoffen aus dieser Gruppe ausgewählt werden, um die physikalischen oder chemischen Eigenschaften der Matrix wie z.B. die Viskosität, die mechanische Festigkeit oder die Auflöseeigenschaften des polymeren Gerüstes auf den Anwendungszweck abzustimmen. So können beispielsweise mit Zusätzen von Dextranen, modifizierten Stärken, Zuckern und insbesondere Mannit erfindungsgemäß Pellets hergestellt werden, die sich spontan in kaltem Wasser vollständig auflösen.

Besonders geeignet als Weichmacherzusätze sind Stoffe wie z.B. Sorbitol, die nach der Trocknung bei Raumtemperatur fest sind. Erstaunlicherweise bildet die Matrix solcher Pellets nach der Lyophilisation eine feste bis halbfeste Struktur aus, die nach Kontakt mit wäßrigem Medium bzw. unter physiologischen Bedingungen eine bioadhäsive und hochviskose Eigenschaft im Sinne der Erfindung ergibt.

Werden Feststoffe verarbeitet, so können diese entweder in der flüssigen Matrixmasse gelöst oder suspendiert werden, z.B. Trockenextrakte.

Handelt es sich um flüssige, lipophile Extrakte (fette oder ätherische Öle), so werden diese in der flüssigen Matrixmasse emulgiert. Dabei können vorteilhaft die Tensideigenschaften der Matrixbestandteile, wie z.B. Gelatine oder Kollagenhydrolysat ausgenutzt werden, sodaß in vielen Fällen ohne Zusatz von Emulgator gearbeitet werden kann. Dies ist ein wesentlicher Vorteil beim peroralen Einsatz wie auch beim Einsatz auf empfindlicher oder verletzter Haut bzw. bei kosmetischen Anwendungen. Mikroemulsionen können mit der Matrixmasse vermischt ebenfalls pelletiert werden.

Durch einfache oder komplexe Koazervation verkapselte fette oder ätherische Öle sowie durch Sprühtrocknungsverfahren verkapselte ätherische Öle können in der erfindungsgemäßen Matrixmasse verarbeitet werden. Es können auch in der gelösten Matrixmasse selbst Mikrokapseln oder Koazervate erzeugt werden, die dann zusammen mit der Matrixmasse zu Pellets geformt werden und in der Matrix inkorporiert Mikrokapseln enthalten. Ebenso kann mit Nanokapseln verfahren werden.

Weiterhin kann es für kosmetische Zwecke erwünscht sein, den beschriebenen Matrixmassen lipophile Bestandteile, wie z.B. Phospholipide zur Ausbildung von Liposomen zuzusetzen.

In Ausnahmefällen können die Pflanzeninhaltsstoffe selbst, insbesondere nach Aufkonzentrierung, als Gerüstbildner für die erfindungsgemäße Pelletherstellung dienen.

Selbstverständlich eignen sich die erfindungsgemäßen Mischungen zu einer sofortigen Abfüllung in flüssiger Form nach dem unter a) beschriebenen Verfahrensschritt zur Ausformung von Behältnissen, wie z.B. Formen, Weichgelatinekapseln sowie geeignete andere Umhüllungen.

In einer Ausführungsform des unter b) beschriebenen Verfahrensschrittes wird die beschriebene Matrixmasse zur Ausrundung (Formgebung) und Schockfrostung in ein Tauchbad im Bereich von -108°C bis -210°C ein. Als tiefkalte und inerte Flüssigkeit wird vorzugsweise flüssiger Stickstoff eingesetzt, der die Bestandteile der Pellets nicht verändert. In der tiefkalten Flüssigkeit bilden sich runde Formkörper (Pellets), die nach der Trocknung eine mechanisch stabile Matrix ausbilden. Die Formgebung erfolgt über ein geeignetes Dosiersystem. Jeder diskrete Tropfen nimmt dabei einerseits bereits während des freien Falls, andererseits im Tauchbad durch die um ihn entstehende Gashülle bzw. die Grenzflächenspannung System/Gas Kugelgestalt an, bevor ein vollständiges Ausfrieren erfolgt. Gerade dieses schnelle, aber dennoch kontrolliert steuerbare Gefrieren fixiert den gegebenen Zustand des Systems augenblicklich, d.h. es können keine Inhaltsstoffe des Pflanzenextraktes in das umgebende Medium diffundieren, gelöste Bestandteile können nicht mehr auskristallisieren, Suspensionen können nicht mehr sedimentieren, Emulsionen nicht brechen, thermisch empfindliche oder feuchtigkeitsempfindliche Bestandteile des Pflanzensaftes werden cryokonserviert, das Trägergerüst kann nicht zusammenschrumpfen usw. Das Herstellungsverfahren mit einem inerten Flüssiggas hat also keine nachteilige Beeinflussung oder Veränderung des Produkts zur Folge. Von besonderem Vorteil ist somit der Erhalt der gewünschten Eigenschaften. Weiterhin arbeitet das Verfahren lösungsmittelfrei, belastet die Umwelt nicht und kann unter Sterilbedingungen durchgeführt werden.

Als Dosiersysteme eignen sich alle Vorrichtungen, die diskrete, gleichmäßige Tropfen vorherbestimmbarer Größe erzeugen können, z.B. pipettenartige Tropfvorrichtungen, geeignete Sprüh- oder Zerstäubungsdüsen mit Dosierpumpen.

Weiterhin können für das erfindungsgemäße Verfahren Dosiervorrichtungen mit Einstoffdüsen, die das zu tropfende Gut getaktet oder intermittierend ausstoßen, verwendet werden.

Eine weiterhin bevorzugte Ausführungsform des erfindungsgemäßen Verfahrens setzt das von Messer Griesheim GmbH entwickelte Cryopel®-Verfahren (basierend auf DE-OS 37 11 169) ein. In Verbindung mit einer Tauchfrostanlage, der Cryopel®-Anlage, ist die apparative Umsetzung des erfindungsgemäßen Verfahrens in den industriellen Maßstab besonders einfach. Diese Anlage, die mit flüssigem Stickstoff betrieben werden kann, zeichnet sich besonders durch ihre Wirtschaftlichkeit aus. Diese Anlage ist auch für Sterilherstellung geeignet. Kontinuierliche Arbeitsweise bei geringem Wartungs- und Reinigungsaufwand ermöglicht die wirtschaftliche Umsetzung des erfindungsgemäßen Verfahrens in den industriellen Maßstab.

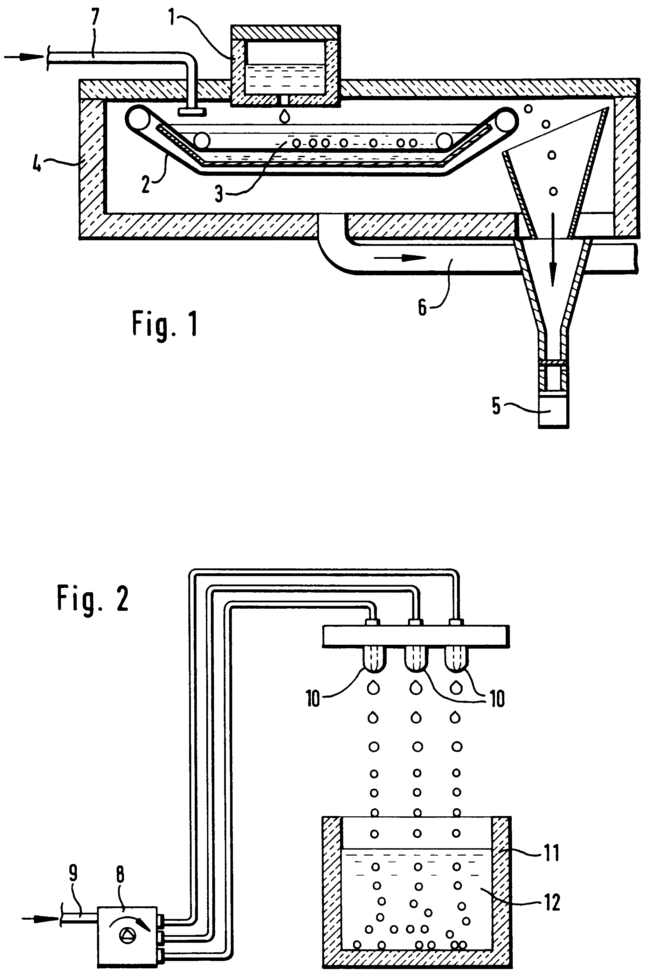

In Fig. 1 ist das von Messer Griesheim GmbH entwickelte Cryopel®-Verfahren schematisch dargestellt. Die erfindungsgemäße Pflanzenextrakt-Matrixmasse wird aus der beheizbaren Eintragsvorrichtung 1 über kalibrierte Düsen in das Flüssigstickstoffbad 3 bei -196°C eingetropft und unter gleichzeitiger Schockfrostung zu runden Pellets geformt. Über das kontinuierlich laufende Transportband 2 wird das gefrorene Produkt über die Vorrichtung 5 ausgetragen. Die Dosierung des Flüssigstickstoffes erfolgt über die Zuleitung 7 und das entstehende Stickstoffgas entweicht über die Leitung 6. Die Isolierung 4 umschließt das gesamte System.

In Fig. 2 ist eine schematische Darstellung eines Verfahrens gezeigt, bei der über eine regelbare Dosierpumpe 8 die kalte bzw. auf max. 50°C erwärmte Pflanzenextrakt-Matrixmasse über die Zuleitung 9 kontinuierlich über die beheizbaren Tropfdüsen 10 in die Isolierwanne 11 mit Flüssigstickstoff 12 eintropft. Die schockgefrosteten Pellets werden chargenweise entnommen. Mit dieser Vorrichtung lassen sich hochviskose Massen verarbeiten.

Sollte das zu verarbeitende System nicht ausreichend fließ- bzw. tropffähig sein, kann z.B. weiterer Wasserzusatz von 1-10 Gew.% erfolgen, die Verarbeitungstemperatur erhöht werden oder aber auch Druck bei der Dosierung zur Anwendung kommen. Im umgekehrten Falle (System zu niedrigviskos) ist analog Unterdruck anzuwenden. Auf diese Weise gewährleistet man gleichmäßige Bildung, wie auch Abriß der einzelnen Tropfen.

Die Verarbeitungstemperatur kann in weiten Bereichen variiert werden, soll aber im Falle von speziellen Pflanzenextrakten, wie z.B. Aloe Vera, unterhalb von 50°C, um eine thermische Belastung der Inhaltsstoffe zu vermeiden.

Somit können beispielsweise mit einer Cryopel®-Dosiervorrichtung Massen, deren Viskosität sich in einem weiten Bereich bewegt, z.B. 1x10-3 bis 12,5 Pa x s (Pascalsekunden), problemlos dosiert werden.

Weitere tiefkalte Flüssigkeiten, die sich für das erfindungsgemäße Verfahren eignen, können z.B. flüssige Edelgase wie Argon sein.

In Abhängigkeit vom gewählten Dosiersystem kann eine Korngrößeneinheitlichkeit von über 70% erreicht werden, die sich durch Klassieren noch zusätzlich erhöhen läßt.

Durch Klassieren abgetrennte Anteile können erneut in den flüssigen Zustand überführt und wieder pelletiert werden, so daß ein verlustfreies Arbeiten gewährleistet ist.

In einer Ausführungsform des beschriebenen Verfahrensschrittes werden die Pellets getrocknet, wobei sich zwei Verfahrensvarianten ergeben.

### Variante A:

Die bei -196°C gefrorenen Pellets werden in eine Gefriertrocknungsanlage überführt. Dabei werden Temperaturen von 15°C unterhalb des Sublimationspunktes von Wasser bei einem Druck von 0,1 Pa bis 103 Pa (0,001 bis 1,03 mbar) gewählt. Der Trocknungsvorgang, der in einer herkömmlichen Gefriertrocknungsanlage (Kondensatortemperatur -40°C) bei -25°C und 33 Pa (0,33 mbar) in der Primärtrocknung unter Sublimation des durch die Schockfrostung amorph gefrorenen Wassers aus der Matrix abläuft, führt nach Sekundärtrocknung (Desorption) zu einem Endprodukt mit einem hochporösen Netzwerk. Solche Pellets sind gegenüber herkömmlich gefriergetrockneter Ware besonders leicht löslich und sind bevorzugt zur Entwicklung von Instantzubereitungen geeignet.

### Variante B:

Die gefrorenen Pellets werden aufgetaut und konventionell getrocknet. Hierbei kann vorteilhaft zur Beschleunigung des Trocknungsvorgangs und zur Einhaltung von niedrigen Temperaturen unter Vakuum (3.000-5.000 Pa (30-50 mbar)) gearbeitet werden. Es können Trocknungstemperaturen von bis zu 50°C gewählt werden, wobei die Temperatur während des Trocknungsvorganges in der Pelletmatrix aufgrund der Verdampfungsenthalpie der Flüssigkeit nicht über 30°C ansteigt.

Für konventionell getrocknete Pellets (Variante B) sind sol-gel-bildende Substanzen für die Matrix notwendig, die in Solform tropffähig sind und nach der Cryopelletierung bzw. nach dem Auftauen ein Gel ausbilden, das nach der Trocknung stabil ist. Ein Zusatz von Weichmachern fördert den Erhalt von einheitlich runden Formkörpern. So hergestellte Pellets zeichnen sich durch eine kostengünstige Herstellung aus und können sowohl im kosmetischen als auch pharmazeutischen Bereich eingesetzt werden.

Das erfindungsgemäße Verfahren selbst ist gegenüber dem Stand der Technik insgesamt gesehen wartungsarm und wirtschaftlich durchzuführen.

Die erfindungsgemäßen Pellets können sowohl für pharmazeutische Zwecke als auch für perorale sowie kosmetische Zwecke geeignet sein.

Pharmazeutische Anwendungen sind beispielsweise:
- Einzeldosierte perorale Arzneiform (Pellets von 2-12 mm)
- Pellets können auch direkt in Hartgelatinekapseln oder Beuteln abgefüllt werden.
- Als Grundlage für die Herstellung von Tabletten, Dragees etc.
- Die Pellets eignen sich hervorragend zur Direkttablettierung. Aufgrund der hohen erzielbaren Korngrößengenauigkeit treten keine Dosierprobleme auf.
- Instanttees
- In Beutel abgefüllt, können Pellets zur Bereitung von health-care Trinklösungen (Instant-Zubereitung) angeboten werden. Bei Verwendung von Pflanzenproteinen, Pflanzenproteinhydrolysaten, Kollagenhydrolysaten oder Gelatine mit einem Maximum der Molekulargewichtsverteilung von einigen Hundert D bis unterhalb 10⁵ D lösen sich die erfindungsgemäßen Pellets in Wasser von Raumtemperatur innerhalb weniger Sekunden auf. Es sind auch Mischungen von verschiedenen Pflanzenextrakten oder mit anderen Wirkstoffen in dieser Form möglich.

Die Kombination von Aloe Vera mit Arzneistoffen bzw. Wirkstoffen, die in Diätetika (health care) enthalten sind, kann ferner zu einer Verträglichkeitssteigerung dieser Substanzen, insbesondere bei der innerlichen Anwendung beitragen. So kann z.B. die Magenschleimhautreizung bzw. Irritation von Acetylsalicylsäure durch schleimhautprotektive Wirkung der Bestandteile des Aloe Vera Saftes wirkungsvoll vermindert werden.
- Balneotherapeutika, Inhalationsmittel zum Auflösen in heißem Wasser
- Herstellung von Salben, Cremes, Gelen etc. für Wundbehandlungen, bei Verbrennungen und Verätzungen etc.
- Herstellung von Wundpflastern, Wundpudern etc.
- steril hergestellte Wirkstoffpellets als Wundinserte

Kosmetische Anwendungen sind beispielsweise:
- Herstellung von Cremes, Instantcremes, Feuchtigkeitsemulsionen, Sonnenschutzmitteln, Mittel gegen Sonnenbrand, Shampoos, Zahnpasten, Seifen, Badezusätze, Gesichtswässer
- direkte Verwendung von Pellets zur Zubereitung von Gesichtsmasken, Pudern u.a.
- Verwendung in Kosmetika in gelöster oder halbfester Form
- Verwendung in Kosmetika in Kombination mit anderen Wirkstoffen

Durch die große Variabilität der Rezepturmassen und der beschriebenen Herstellungsverfahren können die Eigenschaften der erfindungsgemäßen Pellets sehr einfach auf den gewünschten Verwendungszweck abgestimmt werden.

Spezielle Matrixausbildung ermöglicht den direkten Einsatz von Pellets in fester und halbfester Form, wobei die Auflösung bei der Applikation erfolgt.

Durch Variation der Bloomzahl der erfindungsgemäß eingesetzten Gelatine können nicht nur Eigenschaften, wie z.B. die Auflösegeschwindigkeit der erfindungsgemäßen Pellets gesteuert werden, sondern eine gewünschte Viskosität einer daraus gewonnenen wässrigen Lösung kann ebenfalls je nach Anwendungszweck eingestellt werden.

Solche Pellets haben eine Reihe von Vorteilen: Gegenüber auf herkömmliche Weise hergestellten Flüssig-oder Trockenextrakten bleiben die Pflanzeninhaltsstoff in unkonservierter Form unverändert.

Im Falle von Weichmacherzusätzen besitzen sie ein unverwechselbares Aussehen und sind zudem sehr angenehm einzunehmen. Unangenehmer Geschmack wird bereits durch die Matrixbestandteile selbst überdeckt.

Sie ermöglichen eine alkoholfreie Darreichungsform für Pflanzenextrakte oder können eingesetzt werden als homöopathische Globuli. Gegenüber Weichgelatinekapseln ist kein Ausfließen des Wirkstoffes möglich. Flüchtige ätherische Öle werden in eine feste Form gebracht. Sie besitzen im Gegensatz zu handelsüblichen Lösungen und Tinkturen geringes Gewicht und sind als einzeldosierte Arzneiformen leicht schluckbar.

Die nachfolgenden Beispiele sollen die Erfindung näher erläutern:

### Beispiel 1: Pellets als Badezusatz für ein medizinisches Bad bei rheumatischen Beschwerden (pharmazeutische Anwendung)

2,5 kg Gelatine 150 Bloom
1,0 kg Glycerin
6,5 kg Wasser
375 g Wacholderbeerenöl (ätherisches Öl)

Die Gelatine wird mit der Glycerin-Wasser-Mischung bei Raumtemperatur 30 Min vorgequollen und dann bei 60°C aufgelöst. Nach Zugabe des ätherischen Öles aus Wacholderbeeren wird mit einem Ultra-Turrax homogenisiert und die entstandene Emulsion über die Dosiervorrichtung nach Fig 2 in flüssigen Stickstoff mit einer Temperatur von - 196°C eingetropft. Die Pellets werden bei 20°C 24 Stunden luftgetrocknet und in Gefäße abgefüllt.

20 g dieser Pellets als Zusatz zu einem Vollbad lösen sich vollständig im warmen Wasser auf und setzen dabei das ätherische Öl frei. Man erhält ein medizinisches Bad gegen rheumatische Beschwerden.

Vorteilhaft gegen Muskelschmerzen ist auch eine Mischung aus 10 g dieser Pellets mit 10g auf die gleiche Weise hergestellten Rosmarinölpellets.

Zur Herstellung eines Beruhigungsbades kann Melissenöl eingesetzt werden.

Zur Herstellung von Inhalationspellets wird Oleum Pini Pumilionis eingesetzt und die Pellets in heißen Wasser vor der Inhalation aufgelöst.

### Beispiel 2:Vitamin E-Emulsionspellets, gefriergetrocknet zur Verarbeitung in einer Schutzcreme (kosmetische Anwendung)

0,15 kg aus Weizenkeimöl gewonnenes Vitamin E
1,0 kg Kollagenhydrolysat, Molekulargewicht 13.000 - 18.000 g/Mol
9 kg Wasser

Das Kollagenhydrolysat wird bei Raumtemperatur im Wasser gelöst und unter Homogenisieren mit einem Ultra-Turrax wird das flüssige Vitamin E. zugegeben. Die entstandene Emulsion wird über die Dosiervorrichtung aus Fig. 2 in flüssigen Stickstoff von -196°C getropft und so schockgefrostet. Anschließend wird das Wasser aus den Pellets durch Gefriertrocknung entfernt.

Die getrockneten Pellets werden als "festes" Vitamin E in folgende Schutzcreme eingearbeitet:

### Fettphase:

| | |
|---|---|
| Tegomuls^{R} 90S | 2,5 kg |
| Sojaöl | 5,0 kg |
| Kakaobutter | 1,5 kg |
| Cetylalkohol | 1,5 kg |

### Wasserphase:

| | |
|---|---|
| dest. Wasser | 3,0 kg |

### Wirkstoff:

Vitamin E 30 g
entsprechen 230 g Vit E-Emulsionspellets

Die Pellets werden in 1,8 l Wasser emulgiert.

Die Bestandteile der Fettphase werden bei 65°C geschmolzen, 1,2 kg des auf dieselbe Temperatur erwärmten Wassers unter Rühren homogen vermischt. Nach Abkühlen der Creme auf 30°C wird die Vitamin E-Kollagenhydrolysat-Emulsion eingerührt.

### Beispiel 3: Echinacea-Pellets, einzelndosierte Arzneiform

| | |
|---|---|
| Echinacea Urtinktur | 2,16 kg |
| Kollagenhydrolysat, mittleres Molekulargewicht 3.000 g/Mol | 0,50 kg |
| Aqua dest. | 0,50 kg |

Das Kollagenhydrolysat wird im Wasser bei Raumtemperatur gelöst und mit der Urtinktur vermischt. Der Ethanol wird aus dem Ethanol-Wasser-Gemisch bei 40°C unter einem Vakuum von 5.000 Pa (50 mbar) in einem einstufigen Vakuumverdampfer entfernt.

Die echinacea-haltige Lösung wird über eine Dosiervorrichtung nach Fig.2 in flüssigen Stickstoff von -196°C eingetropft und so die Pellets geformt. Diese werden anschließend einer Gefriertrocknung mit einer Primärtrocknung bei -50°C und 5 Pa (0,05mbar) und einer Sekundärtrocknung bei 22°C unterzogen.

Nach der Trocknung haben die Echinacea-Pellets einen Durchmesser von 5 mm. Die Einnahme von 3x1 Pellet pro Tag entspricht der Dosierung als vorbeugendes Mittel gegen Erkältungskrankheiten.

### Beispiel 4: Echinacea-Weichgelatinepellets:

| | |
|---|---|
| Gelatine (140 Bloom) | 250 g |
| Glycerin | 100 g |
| Ecinacea Frischpflanzensaft (Preßsaft) | 5000,0 g |

Man läßt die Gelatine in der Mischung aus Frischpflanzensaft und Glycerin 30 min. quellen, erwärmt auf 40°C und entfernt am einstufigen Verdampfer bei 40°C und einem Vakuum von 5.000 Pa (50 mbar) soviel Wasser, daß die Masse noch fließfähig ist. Die Masse wird über eine Dosiervorrichtung nach Fig 2 wie in Beispiel 1 cryopelletiert und unter den angegebenen Bedingungen getrocknet. Die Pellets mit einem Ducrchmesser von 3,5 mm werden in einen Dosierspender abgefüllt. Als Prophylaktikum entnimmt man pro Einzeldosis 5 Pellets.

### Beispiel 5: Rutinsuspension

| | |
|---|---|
| Gelatine 140 Bloom | 200 g |
| Glycerin | 150 g |
| Aqua dest | 650g |
| Rutin | 87,5 g |

Aus Gelatine, Glycerin und Wasser wird wie in Beispiel 1 eine Lösung hergestellt und das Rutin in Pulverform suspendiert. Es wird wie in Beispiel 1 pelletiert und getrocknet. 5 Pellets mit einem Durchmesser von 3,5 mm enthalten eine Dosis von 50 mg Rutin.

### Beispiel 6: Aloe Vera Saft

150 g Kollagenhydrolysat, mittleres Molekulargewicht: 3.000 g/mol
3000 g Aloe Vera Saft, Feststoffkonzentration 0,6% (g/g)

Die frisch gewonnene Filets von Aloe Vera-Blättern werden homogenisiert, gereinigt und filtriert. Das Kollagenhydrolysat wird unter Rühren in dem so gewonnenen und gekühlten Aloe Vera Saft gelöst. Anschließend werden aus der Lösung über die Cryopel^{R}-Eintragsvorrichtung in einem Tauchbad mit flüssigem Stickstoff bei -196°C Pellets geformt.

Die schockgefrosteten, runden Formkörper werden in einer Gefriertrocknungsanlage mit einer Primärtrocknung bei -50°C und 5 Pa (0,05 mbar) und einer Sekundärtrocknung bei 22°C getrocknet.

Man erhält Pellets mit einem Durchmesser von 4 mm und einem Gehalt an Aloe Vera von 10,7% (g/g Trockensubstanz). Durch Klassieren wird eine Korngrößengenauigkeit von 78% bestimmt.

Die Pellets lösen sich in Wasser von Raumtemperatur innerhalb von 20 Sekunden vollständig auf.

### Beispiel 7:

100 g Kollagenhydrolysat, mittleres Molekulargewicht: 3.000 g/mol
50 g Mannit
50 g Weizenproteinhydrolysat, Molekulargewicht < 5.000 g/mol
2000 g Aloe Vera Saft, Feststoffkonzentration 0,6%

In dem Aloe Vera Saft, das wie in Beispiel 6 aufgearbeitet ist, wird das Kollagenhydrolysat, das Weizenproteinhydrolysat und Mannit kalt aufgelöst und wie in Beispiel 6 Pellets hergestellt. Man erhält Pellets mit einem Durchmesser von 3 mm und einem Aloe Vera-Feststoffanteil von 5,7% (g/g). Diese Pellets können in Drangen- oder Maracujasaft aufgelöst als Trinklösung verwendet werden.

### Beispiel 8:

200 g Kollagenhydrolysat, mittleres Molekulargewicht: 3.000 g/mol
4000 g Aloe Vera Saft, 10-fach-Konzentrat

Das in Beispiel 6 gewonnene Aloe Vera Saft wird bei 40°C unter Vakuum bei 5.000 Pa (50 mbar) mittels einstufigem Vakuumverdampfers auf das 10-fache konzentriert. Das Kollagenhydrolysat wird in dem Saft gelöst und nach Kurzzeit-Pasteurisierung lyophilisierte Pellets hergestellt. Man erhält runde Formkörper mit einem Durchmesser von 4,5mm und einem Aloe Vera-Feststoffanteil von 54,5% (g/g).
Die Pellets lösen sich in Wasser von Raumtemperatur innerhalb von 40 Sekunden auf.

5 g dieser Pellets in 100 ml sterilem Wasser aufgelöst, ergeben eine wirksame Instant-Rezeptur gegen Sonnenbrand.

### Beispiel 9:

### Einarbeiten der erfindungsgemäßen Pellets in eine Nachtcreme

### a) Pelletherstellung

300 g Kollagenhydrolysat, mittleres Molekulargewicht: 13.000- 18.000 g/mol
4000 g Aloe Vera Saft, Feststoffkonzentration 0,6%

Wie in Beispiel 6 beschrieben, werden lyophilisierte Pellets hergestellt mit einem Feststoffanteil von Aloe Vera von 7,4% (g/g).

### b) Rezeptur der Nachtcreme

### Fettphase:

200 g Tegomuls 90S
750 g Avocadoöl

### Wasserphase:

200 g natives Kollagen (3%-ige Lösung, Molekulargewicht 300.000 g/mol)
30 g Elastin
32 g Aloe Vera Pellets aus a)
3000 g frisch destilliertes Wasser

Die Fettphase wird bei 70°C geschmolzen. Das Wasser wird ebenfalls auf 70°C erhitzt und das Elastin darin gelöst. Die erhaltene wässrige Lösung wird in der Fettphase homogenisiert. Die Cremegrundlage wird auf 35°C abgekühlt. In der kalten Kollagenlösung werden die Aloe Vera Pellets aufgelöst und in der Cremegrundlage homogen dispergiert.

### Beispiel 10:

400 g handelsübliche Gelatine (170 Bloom)
300 g Glycerin (85%)
1300 g Aloe Vera Saft, Feststoffkonzentration 0,5% (g/g)

Das Gelatinepulver wird zu dem frisch gewonnenen und homogenisierten Aloe Vera Saft gegeben und ca. 45 Minuten vorgequollen. Die Mischung wird anschließend bei 40°C vollständig aufgelöst und das Glycerin homogen zugemischt.

Anschließend wird über die in Fig. 2 dargestellte Anlage die 40°C warme Lösung über die Pumpe in das Tauchbad mit flüssigem Stickstoff dosiert und Pellets geformt. Die tiefgefrorenen, runden Formkörper werden aufgetaut und bei ansteigender Temperatur zwischen 25°C und 40°C getrocknet. Die Pellets weisen einen Restfeuchtegehalt von 10% auf und sind lagerstabil.

Die so hergestellten Pellets können in ein handelsübliches Hydrogel (z.B. Polyacrylatgel) eingearbeitet werden. In dem Hydrogel quellen die Pellets nach 10 bis 15 Min. bis zum 2-fachen ihrer ursprünglichen Größe auf und bilden gelförmige, leicht schmelzbare Formkörper, die sich nach Auftragen auf der Haut auflösen.

Alternativ können die Pellets auch ohne Trocknung und ohne Zwischenlagerung direkt in das Hydrogel gegeben werden.

## Patentansprüche

1. Pflanzenwirkstoffextrakt enthaltende kryopelletierte Formkörper, gekennzeichnet durch eine Dispersion des Pflanzenextraktes in einer Matrix, die vorwiegend einen Gerüstbildner aus hydrophilen Makromolekülen umfaßt, ausgewählt aus der Gruppe bestehend aus: Kollagen, Gelatine, fraktionierte Gelatine, Kollagenhydrolysate, Gelatinederivate, Elastinhydrolysate, Pflanzenproteine, Pflanzenproteinhydrolysate; sowie deren Mischungen.

2. Formkörper nach Anspruch 1, dadurch gekennzeichnet, daß das hydrophile Makromolekül ein thermoreversibler Sol-Gel-Bildner ist.

3. Formkörper nach Anspruch 1, dadurch gekennzeichnet, daß die Matrix einen zusätzlichen Gerüstbildner aus der Gruppe: Albumine, Pflanzenproteine, Pflanzenproteinhydrolysate, Agar-Agar, Gummi arabicum, Pektine, Traganth, Xanthan, natürliche sowie modifizierte Stärken, Dextrane, Dextrine, Maltodextrin, Chitosan, Alginate, Cellulosederivate, Dextran, Zucker, Glycin, Lactose, Sorbit, Mannit oder Polyvinylpyrrolidon, umfaßt.

4. Formkörper nach Anspruch 3, dadurch gekennzeichnet, daß der Gehalt der Matrix an zusätzlichem Gerüstbildner weniger als 50 Gew.-% beträgt.

5. Formkörper nach Anspruch 1, gekennzeichnet durch einen pharmazeutisch akzeptablen Hilfs- oder Trägerstoff für die Matrix.

6. Formkörper nach Anspruch 1, gekennzeichnet durch einen Gehalt an Pflanzenextrakt von 0,1-98 Gew.-% (berechnet als Trockensubstanz).

7. Formkörper nach Anspruch 1, dadurch gekennzeichnet, daß der Pflanzenextrakt ausgewählt ist aus der Gruppe bestehend aus: feste Pflanzenextrakte, flüssige Pflanzenextrakte, hydrophile Pflanzenextrakte, lipophile Pflanzen-extrakte, einzelne Pflanzeninhaltsstoffe; sowie deren Mischungen.

8. Formkörper nach einem der Ansprüche 1-7, dadurch gekennzeichnet, daß der Pflanzenextrakt Aloe Vera Saft ist.

9. Formkörper nach Anspruch 1, dadurch gekennzeichnet, daß sie als Lyophilisat vorliegen.

10. Formkörper nach Anspruch 1, dadurch gekennzeichnet, daß er schnellauflösend ist; und daß die Matrix im wesentlichen aus einem Pflanzenprotein, Pflanzenproteinhydrolysat, Kollagenhydrolysat, einem kaltwasserlöslichen Gelatinederivat oder Gelatine mit einem Maximum der Molekulargewichtsverteilung unterhalb 10⁵ D besteht.

11. Formkörper nach Anspruch 1, dadurch gekennzeichnet, daß die Matrix die Weichmacher Glycerin oder Sorbitol und Geschmackskorrigentien von 1-50 Gew.-% (bezogen auf die zu verarbeitende Masse) enthält.

12. Formkörper nach Anspruch 11, dadurch gekennzeichnet, daß er in fester, halbfester oder gelförmiger Form vorliegt.

13. Formkörper nach Anspruch 12, dadurch gekennzeichnet, daß er als Pellet vorliegt.

14. Formkörper nach einem der Ansprüche 2-13, dadurch gekennzeichnet, daß der Sol-Gel-Bildner eine Gelatine mit einem Maximum der Molekulargewichtsverteilung oberhalb 10⁵ D ist.

15. Verfahren zur Herstellung von Pflanzenwirkstoffextrakt enthaltenden Formkörper, dadurch gekennzeichnet, daß man
a) einen Gerüstbildner aus hydrophilen Makromolekülen ausgewählt aus der Gruppe bestehend aus: Kollagen, Gelatine, fraktionierter Gelatine, Kollagenhydrolysaten, Gelatinederivaten Pflanzenproteinen, Pflanzenproteinhydrolysaten, Elastinhydrolysaten; sowie deren Mischungen,
mit Pflanzenwirkstoffextrakten vermischt, welche ausgewählt werden aus der Gruppe bestehend aus:
hydrophilen flüssigen Pflanzenextrakten, wäßrigen Extrakten, alkoholischen Extrakten; sowie deren Mischungen; und
b) die erhaltene Mischung aus Gerüstbildner und Pflanzenextrakt in ein tiefkaltes inertes verflüssigtes Gas eintropft und damit Formkörper formt.

16. Verfahren nach Anspruch 15, dadurch gekennzeichnet, daß man als Gerüstbildner einen Sol-Gel-Bildner einsetzt.

17. Verfahren nach Anspruch 15 und/oder 16, dadurch gekennzeichnet, daß man als Formkörper einen Pellet herstellt.

18. Verfahren nach einem der Ansprüche 15-17, dadurch gekennzeichnet, daß man als Pflanzenextrakt Aloe Vera Saft einsetzt.

19. Verfahren nach Anspruch 15-17, dadurch gekennzeichnet, daß man die Pellets nach Stufe b) direkt in eine Creme- oder Hydrogelgrundlage einarbeitet.

20. Verfahren nach Anspruch 17, dadurch gekennzeichnet, daß man die Pellets gefriertrocknet.

21. Verfahren nach Anspruch 15, dadurch gekennzeichnet, daß man in Stufe a) eine Lösung aus dem Gerüstbildner herstellt und mit dem hydrophilen flüssigen Pflanzenextrakt vermischt.

22. Verfahren nach Anspruch 15, dadurch gekennzeichnet, daß man im Falle von alkoholischen Extrakten das organische Lösungsmittel entfernt.

23. Verfahren nach Anspruch 15, dadurch gekennzeichnet, daß man in Stufe a) die Lösung aufkonzentriert.

24. Verfahren nach Anspruch 15, dadurch gekennzeichnet, daß man einen lipophilen Extrakt in Stufe a) in der Matrixmasse emulgiert, oder einen festen Pflanzenextrakt suspendiert oder löst.

25. Verfahren nach Anspruch 15, dadurch gekennzeichnet, daß man die erhaltene Mischung in flüssigen Stickstoff eintropft.

26. Verfahren nach Anspruch 15, dadurch gekennzeichnet, daß man die Dispersion aus Gerüstbildner und Pflanzenexrakt mit einem zusätzlichen Gerüstbildner aus der Gruppe: Albumin, Agar-Agar, Gummi arabicum, Pektine, Traganth, Xanthan, natürliche sowie modifizierte Stärken, Dextrane, Dextrine, Maltodextrin, Chitosan, Alginate, Cellulosederivate, Dextran, Zucker, Glyzin, Lactose, Mannit oder Polyvinylpyrrolidon versetzt.

27. Verfahren nach Anspruch 15, dadurch gekennzeichnet, daß der Mischung aus Gerüstbildner und Pflanzenextrakt die Weichmacher Glycerin, Sorbit oder deren Mischungen von 1-50 Gew.-% (bezogen auf die zu verarbeitende Masse) zugesetzt werden.

28. Verfahren nach Anspruch 15, dadurch gekennzeichnet, daß als Gerüstbildner Gelatine mit einem Maximum der Molekulargewichtsverteilung oberhalb 10⁵ D bei maximal 60°C mit dem Pflanzenextrakt vermischt wird.

29. Verfahren nach Anspruch 28, dadurch gekennzeichnet, daß flüssiger Aloe Vera Saft bei maximal 40°C mit Gelatine vermischt wird.

30. Verfahren nach einem der Ansprüche 15-29, dadurch gekennzeichnet, daß man die Formkörper bei maximal 50°C trocknet.

31. Verwendung der Formkörper nach Anspruch 1 zur Herstellung einer pharmazeutischen Zubereitung.

32. Verwendung der Formkörper nach Anspruch 1 zur Herstellung einer peroralen Zubereitung.

33. Verwendung der Formkörper nach Anspruch 1 zur Herstellung einer kosmetischen Zubereitung.

34. Pharmazeutische Zubereitung, enthaltend Formkörper nach Anspruch 1.

35. Nahrungsmittelzubereitung (health care), enthaltend Formkörper nach Anspruch 1.

36. Kosmetische Zubereitung, enthaltend Formkörper nach Anspruch 1.

37. Aloe Vera Saft enthaltende kryopelletierte Formkörper, gekennzeichnet durch eine Dispersion des Aloe Vera Saftes in einer Matrix, die vorwiegend Gerüstbildner aus hydrophilen Makromolekülen, ausgewählt aus der Gruppe bestehend aus: Kollagen, Gelatine, fraktionierte Gelatine, Kollagenhydrolysate, Gelatinederivate, Pflanzenproteine, Pflanzenproteinhydrolysate, Elastinhydrolysate sowie deren Mischungen enthält.

## Claims

1. Cryopelleted shaped article containing plant-derived active substance extract, characterized by a dispersion of the plant extract in a matrix composed predominantly of a skeleton builder of hydrophilic macromolecules selected from the group consisting of: collagen, gelatin, fractionated gelatin, collagen hydrolysates, gelatin derivatives, elastin hydrolysates, plant proteins, plant protein hydrolysates; and their mixtures.

2. Shaped article according to Claim 1, characterized in that the hydrophilic macromolecule is a thermoreversible sol/gel forming agent.

3. Shaped article according to Claim 1, characterized in that the matrix contains an additional skeleton builder from the group consisting of: albumins, plant proteins, plant protein hydrolysates, agar-agar, gum arabic, pectins, tragacanth, xanthan, natural and modified starches, dextrans, dextrins, maltodextrin, chitosan, alginates, cellulose derivatives, dextran, sugars, glycine, lactose, sorbitol, mannitol or polyvinylpyrrolidone.

4. Shaped article according to Claim 3, characterized in that the matrix contains less than 50 % by weight of additional skeleton builder.

5. Shaped article according to Claim 1, characterized by a pharmaceutically acceptable auxiliary or carrier for the matrix.

6. Shaped article according to Claim 1, characterized by a content of 0.1 - 98 % by weight (calculated as dry substance) of plant extract.

7. Shaped article according to Claim 1, characterized in that the plant extract is selected from the group consisting of:
solid plant extracts, liquid plant extracts, hydrophilic plant extracts, lipophilic plant extracts, individual plant constituents; and their mixtures.

8. Shaped article according to any one of Claims 1-7, characterized in that the plant extract is Aloe vera juice.

9. Shaped article according to Claim 1, characterized in that it is in the form of a lyophilisate.

10. Shaped article according to Claim 1, characterized in that it is rapidly dissolving and in that the matrix is composed of essentially a plant protein, plant protein hydrolysate, collagen hydrolysate, a gelatin derivative which is soluble in cold water, or gelatin whose molecular weight distribution has a maximum of below 10⁵ D.

11. Shaped article according to Claim 1, characterized in that the matrix contains 1-50 % by weight (based on the composition to be processed) of the plasticizers glycerol or sorbitol and masking flavours.

12. Shaped article according to Claim 11, characterized in that it is in solid, semi-solid or gel-like form.

13. Shaped article according to Claim 12, characterized in that it is in the form of a pellet.

14. Shaped article according to any one of Claims 2-13, characterized in that the sol/gel forming agent is a gelatin whose molecular weight distribution has a maximum of above 10⁵ D.

15. Process for the preparation of shaped articles containing plant-derived active substance extract, characterized in that
a) a skeleton builder of hydrophilic macromolecules selected from the group consisting of: collagen, gelatin, fractionated gelatin, collagen hydrolysates, gelatin derivatives, plant proteins, plant protein hydrolysates, elastin hydrolysates; and their mixtures,
is mixed with plant-derived active substance extracts selected from the group consisting of:
hydrophilic liquid plant extracts, aqueous extracts, alcoholic extracts; and their mixtures; and
b) the resulting mixture of skeleton builder and plant extract is added dropwise to an intensely cold inert liquefied gas, thus making it into shaped articles.

16. Process according to Claim 15, characterized in that a sol/gel forming agent is employed as skeleton builder.

17. Process according to Claim 15 and/or 16, characterized in that the shaped article prepared is a pellet.

18. Process according to any one of Claims 15-17, characterized in that the plant extract employed is Aloe vera juice.

19. Process according to Claim 15-17, characterized in that the pellets obtained in step b) are incorporated directly into a cream or hydrogel base.

20. Process according to Claim 17, characterized in that the pellets are freeze-dried.

21. Process according to Claim 15, characterized in that a solution of the skeleton builder is prepared in step a) and mixed with the hydrophilic liquid plant extract.

22. Process according to Claim 15, characterized in that the organic solvent is removed in the event that alcoholic extracts are used.

23. Process according to Claim 15, characterized in that the solution is concentrated in step a).

24. Process according to Claim 15, characterized in that a lipophilic extract is emulsified in the matrix, or a solid plant extract is suspended or dissolved in the matrix in step a).

25. Process according to Claim 15, characterized in that the mixture obtained is added dropwise to liquid nitrogen.

26. Process according to Claim 15, characterized in that an additional skeleton builder from the group consisting of: albumin, agar-agar, gum arabic, pectins, tragacanth, xanthan, natural and modified starches, dextrans, dextrins, maltodextrin, chitosan, alginates, cellulose derivatives, dextran, sugars, glycine, lactose, mannitol or polyvinylpyrrolidone is added to the dispersion of skeleton builder and plant extract.

27. Process according to Claim 15, characterized in that 1-50 % by weight (based on the composition to be processed) of the plasticizers glycerol and sorbitol or their mixtures are added to the mixture of skeleton builder and plant extract.

28. Process according to Claim 15, characterized in that gelatin whose molecular weight distribution has a maximum of above 10⁵ D is mixed with the plant extract at not more than 60°C to act as skeleton builder.

29. Process according to Claim 28, characterized in that liquid Aloe vera juice is mixed with gelatin at not more than 40°C.

30. Process according to any one of Claims 15-29, characterized in that the shaped articles are dried at not more than 50°C.

31. Use of the shaped articles according to Claim 1 for the preparation of a pharmaceutical dosage form.

32. Use of the shaped articles according to Claim 1 for the preparation of a peroral dosage form.

33. Use of the shaped articles according to Claim 1 for the preparation of a cosmetic product.

34. Pharmaceutical dosage form containing shaped articles according to Claim 1.

35. Food preparation (health care) containing shaped articles according to Claim 1.

36. Cosmetic product containing shaped articles according to Claim 1.

37. Cryopelleted shaped article containing Aloe vera juice, characterized by a dispersion of the Aloe vera juice in a matrix predominantly composed of skeleton builders of hydrophilic macromolecules selected from the group consisting of: collagen, gelatin, fractionated gelatin, collagen hydrolysates, gelatin derivatives, plant proteins, plant protein hydrolysates, elastin hydrolysates and their mixtures.

## Revendications

1. Corps modelé pastillé à basse température contenant un extrait de substance active végétale, caractérisé par une dispersion de l'extrait végétal dans une matrice qui comprend principalement un produit formateur d'ossature en macromolécules hydrophiles, choisi dans le groupe constitué par le collagène, la gélatine, la gélatine fractionnée, des hydrolysats de collagène, des dérivés de la gélatine, des hydrolysats de l'élastine, des protéines végétales, des hydrolysats de protéines végétales ; ainsi que leurs mélanges.

2. Corps modelé selon la revendication 1, caractérisé en ce que la macromolécule hydrophile est un agent générateur de sol-gel thermoréversible.

3. Corps modelé selon la revendication 1, caractérisé en ce que la matrice comprend un produit formateur d'ossature supplémentaire appartenant au groupe des albumines, des protéines végétales, des hydrolysats de protéines végétales, de la gélose, de la gomme arabique, des pectines, de la gomme adragante, de la gomme xanthane, des amidons naturels et modifiés, des dextranes, des dextrines, de la maltodextrine, du chitosane, des alginates, des dérivés de la cellulose, du dextrane, des sucres, de la glycine, du lactose, du sorbitol, du mannitol ou de la polyvinylpyrrolidone.

4. Corps modelé selon la revendication 3, caractérisé en ce que la teneur de la matrice en produit formateur d'ossature supplémentaire est inférieure à 50% en poids.

5. Corps modelé selon la revendication 1, caractérisé par un adjuvant ou excipient pharmaceutiquement acceptable pour la matrice.

6. Corps modelé selon la revendication 1, caractérisé en ce qu'il contient un extrait végétal à raison de 0,1 à 98% en poids (calculé en substance sèche).

7. Corps modelé selon la revendication 1, caractérisé en ce que l'extrait végétal est choisi dans le groupe constitué par des extraits végétaux solides, des extraits végétaux liquides, des extraits végétaux hydrophiles, des extraits végétaux lipophiles, divers constituants des plantes, ainsi que leurs mélanges.

8. Corps modelé selon l'une des revendications 1 à 7, caractérisé en ce que l'extrait végétal est du jus d'Aloe Vera.

9. Corps modelés selon la revendication 1, caractérisés en ce qu'ils sont présents sous forme de lyophilisat.

10. Corps modelé selon la revendication 1, caractérisé en ce qu'il est à dissolution rapide ; et en ce que la matrice est essentiellement constituée d'une protéine végétale, d'un hydrolysat de protéine végétale, d'un hydrolysat de collagène, d'un dérivé de la gélatine soluble dans l'eau froide ou d'une gélatine ayant un maximum de la répartition des poids moléculaires inférieur à 10⁵ D.

11. Corps modelé selon la revendication 1, caractérisé en ce que la matrice contient du glycérol ou du sorbitol comme plastifiant et de 1 à 50% en poids d'agent de correction du goût (par rapport à la masse à mettre en oeuvre).

12. Corps modelé selon la revendication 11, caractérisé en ce qu'il est présent sous une forme solide, semi-solide ou sous forme de gel.

13. Corps modelé selon la revendication 12, caractérisé en ce qu'il est présent à l'état de pastille.

14. Corps modelé selon l'une des revendications 2 à 13, caractérisé en ce que l'agent générateur de sol-gel est une gélatine ayant un maximum de la répartition des poids moléculaires supérieur à 10⁵ D.

15. Procédé de préparation d'un corps modelé contenant un extrait de substance active végétale, caractérisé en ce que :
a) on mélange un produit formateur d'ossature constitué de macromolécules hydrophiles choisies dans le groupe constitué par le collagène, la gélatine, la gélatine fractionnée, des hydrolysats de collagène, des dérivés de la gélatine, des protéines végétales, des hydrolysats de protéines végétales, des hydrolysats d'élastine ; ainsi que leurs mélanges,
avec des extraits de substances actives végétales, qui sont choisis dans le groupe constitué par :
des extraits végétaux liquides hydrophiles, des extraits aqueux, des extraits alcooliques ; ainsi que leurs mélanges ; et
b) on fait couler goutte à goutte le mélange de produit formateur d'ossature et d'extrait végétal obtenu dans un gaz liquéfié inerte très fortement refroidi et on forme avec celui-ci des corps modelés.

16. Procédé selon la revendication 15, caractérisé en ce qu'on utilise comme produit formateur d'ossature un produit formateur de sol-gel.

17. Procédé selon les revendications 15 et/ou 16, caractérisé en ce qu'on prépare comme corps modelé une pastille.

18. Procédé selon l'une des revendications 15 à 17, caractérisé en ce qu'on utilise comme extrait végétal du jus d'Aloe Vera.

19. Procédé selon les revendications 15 à 17, caractérisé en ce qu'on incorpore directement les pastilles après l'étape b) dans un excipient pour crème ou hydrogel.

20. Procédé selon la revendication 17, caractérisé en ce qu'on lyophilise les pastilles.

21. Procédé selon la revendication 15, caractérisé en ce qu'on prépare dans l'étape a) une solution du produit formateur d'ossature et on la mélange à l'extrait végétal liquide hydrophile.

22. Procédé selon la revendication 15, caractérisé en ce que dans le cas des extraits alcooliques, on élimine le solvant organique.

23. Procédé selon la revendication 15, caractérisé en ce que dans l'étape a), on concentre la solution.

24. Procédé selon la revendication 15, caractérisé en ce qu'on émulsionne un extrait lipophile dans l'étape a) dans la masse de matrice, ou bien on met en suspension ou dissout un extrait végétal solide.

25. Procédé selon la revendication 15, caractérisé en ce qu'on fait couler goutte à goutte le mélange obtenu dans de l'azote liquide.

26. Procédé selon la revendication 15, caractérisé en ce qu'on additionne la dispersion de produit formateur d'ossature et d'extrait végétal d'un produit formateur d'ossature supplémentaire choisi dans le groupe de l'albumine, de la gélose, de la gomme arabique, des pectines, de la gomme adragante, de la gomme xanthane, des amidons naturels ainsi que modifiés, des dextranes, des dextrines, de la maltodextrine, du chitosane, des alginates, des dérivés de la cellulose, du dextrane, des sucres, de la glycine, du lactose, du mannitol ou de la polyvinylpyrrolidone.

27. Procédé selon la revendication 15, caractérisé en ce qu'on ajoute au mélange de produit formateur d'ossature et d'extrait végétal, comme plastifiant, du glycérol, du sorbitol ou leurs mélanges, à raison de 1 à 50% en poids (par rapport à la masse à mettre en oeuvre).

28. Procédé selon la revendication 15, caractérisé en ce qu'on mélange à l'extrait végétal, comme produit formateur d'ossature, des gélatines ayant un maximum de la répartition des poids moléculaires supérieur à 10⁵ D à 60°C au maximum.

29. Procédé selon la revendication 28, caractérisé en ce qu'on mélange avec la gélatine un jus d'Aloe Vera liquide à 40°C au maximum.

30. Procédé selon l'une des revendications 15 à 29, caractérisé en ce qu'on sèche les corps modelés à 50°C au maximum.

31. Utilisation de corps modelés selon la revendication 1 pour la fabrication d'une composition pharmaceutique.

32. Utilisation de corps modelés selon la revendication 1 pour la fabrication d'une préparation perorale.

33. Utilisation de corps modelés selon la revendication 1 pour la fabrication d'une composition cosmétique.

34. Préparation pharmaceutique contenant des corps modelés selon la revendication 1.

35. Préparation d'aliments (health care) contenant des corps modelés selon la revendication 1.

36. Préparation cosmétique contenant des corps modelés selon la revendication 1.

37. Corps modelé pastillé à basse température contenant du jus d'Aloe Vera, caractérisé par une dispersion du jus d'Aloe Vera dans une matrice qui contient principalement des produits formateurs d'ossature en macromolécules hydrophiles choisies dans le groupe constitué du collagène, de la gélatine, de la gélatine fractionnée, des hydrolysats de collagène, des dérivés de la gélatine, des protéines végétales, des hydrolysats de protéines végétales, des hydrolysats d'élastine ainsi que de leurs mélanges.
